# EUROPEAN PATENT SPECIFICATION

(11) **EP 0 319 764 B1**
(45) Date of publication and mention of the grant of the patent: **30.08.1995**
(21) Application number: 88119376.7
(22) Date of filing: 22.11.1988
(51) Int. Cl.: A61M 1/00, A61M 5/14, A61M 25/00

(54) **Connector with injection site**
Verbindungsstück mit Injektionsstelle
Connecteur avec emplacement pour injection

(30) Priority: 07.12.1987 JP 308860/87; 07.12.1987 JP 186267/87 U
(43) Date of publication of application: 14.06.1989
(73) Proprietor: NISSHO CORPORATION, Osaka-shi (JP)
(72) Inventor: Inoue, Yoshifumi, Ikeda-shi Osaka-fu (JP); Yoshioka, Hiroshi, Takatsuki-shi Osaka-fu (JP)
(74) Representative: Türk, Gille, Hrabal, Leifert

(56) References cited:
- EP-A- 0 114 677
- CA-A- 1 105 959
- DE-A- 2 720 470
- FR-A- 1 447 174
- FR-A- 2 322 614
- US-A- 4 512 766
- US-A- 4 673 400

## Description

The present invention relates to a connector with an injection site and applications thereof, and more particularly to a connector with an injection site and applications thereof capable of, when infusing other liquid drug or nutrient fluid together with liquid drug or nutrient fluid to be infused during fluid therapy such as total parenteral nutrition or intravenous hyper alimentation, infusing liquid drug and/or nutrient fluid (hereafter referred to as liquid drug and the like) from another infusion line through an injection site by providing the injection site at a midway of the infusion line; and capable of, when infusing plural kinds of liquid drug and the like using the same intravenous catheter, infusing plural kinds of liquid drug and the like in order through an injection site by providing the injection site at a proximal portion of the intravenous catheter, i.e. at an introducing portion of liquid drug and the like.

When infusing some liquid drug and the like together with other liquid drug and the like to be infused during fluid therapy such as total parenteral nutrition, there are usually used a solution infusion set or catheter (hereafter referred to as infusion tube) having a part called "injection site" at a midway of the infusion route. The other liquid drug and the like can be infused by sticking a needle of other infusion line into a rubber plug in the injection site of the infusion tube and by making the infusion tube communicate with the other infusion line.

In the fluid therapy using an infusion line comprising an infusion tube and an intravenous catheter connected to the infusion tube, the solution infusion is sometimes interrupted for a long period of time. In that case, the infusion tube is detached from the intravenous catheter and a cap with a rubber plug called "injection plug" is put on the proximal portion of the intravenous catheter (a portion to be connected to the infusion tube). When the temporal solution infusion is required during interruption of a solution infusion, a needle of other infusion line is sticked into the rubber plug of the injection plug to make the intravenous catheter communicate with the other infusion line and to infuse solution.

A connector consisting of a tubular member having a fluid inlet and a fluid outlet as well as side tubes is disclosed in FR-A-2 322 614. A plug of elastic material is inserted into one of the side tubes to form an injection site for a needle to be sticked into the plug. The plug is secured in place by a cap being engaged with a connecting means provided on the external wall of the side tube.

In the conventional infusion line using an injection site or an injection plug, however, a needle sometimes pulls out from the rubber plug when a patient under fluid therapy moves or touches the infusion line, since the needle is merely sticked into the rubber plug in the injection site or injection plug. Further there is a danger of infection from the air since a part of the needle is exposed to the air.

DE-A-2 720 470 discloses a catheter assembly comprising a tubular body having a side tube. An elastic material having a small passage for the catheter is inserted into the side tube. The known assembly further comprises a cap having a plug. When the cap is engaged with the side tube, the elastic material is compressed to form a seal between an inner surface of the side tube and the elastic material and the outer surface of the catheter and the elastic material, respectively. The above engagement is carried out by the connection of a pin and a slot.

It is an object of the present invention to provide an injection assembly consisting of an injection needle and a connector having an injection site which prevents the pulling out of the injection needle during fluid therapy and which isolates the needle from the air so that the invasion of bacteria from the injection side is avoided.

It is a further object of the invention to provide an intravenous catheter and an infusion tube both having an injection site which prevents the pulling out of the injection needle during fluid therapy and which isolates the needle from the air so that the invasion of bacteria from the injection side is avoided.

This problem is solved, according to the invention, with the features of claims 1, 5 and 6, respectively.

In accordance with the present invention, there is provided a connector with an injection site comprising a tubular body having one or more fluid inlet and a fluid outlet, wherein a cap or plug made of rubber-like elastic material is fitted into at least one fluid inlet to form an injection site, and a connecting means is provided on the external wall of the injection site or on the external wall of the tubular body near the injection site.

The connector with an injection site of the present invention has a connecting means on the external wall of the injection site or on the external wall of the tubular body near the injection site.

The needle of the infusion line having on the side of the needle a connecting means corresponding to the connecting means of the connector with the injection site is sticked into, for example, the rubber plug of the injection site of the connector, and the connecting means of the connector and that of the infusion line are connected to each other, so that a firm connection is obtained.

The connecting means of the connector and that of the infusion line are so designed as to provide an airtight connection so that an infection from the air is certainly prevented since the needle of the infusion line is isolated from the air and the needle is not pulled out from the rubber plug of the injection site during the use of infusion line.
Fig. 1 is a perspective view of an embodiment of a connector of the present invention;
Fig. 2a is a sectional view taken along the line X-X of Fig. 1;
Fig. 2b is a partially explanatory view showing a connention end of an infusion line to be connected to the connector shown in Fig. 2a;
Fig. 3a is a partially sectional view of another embodiment of a connector of the present invention;
Fig. 3b is a respectively partially sectional view showing a connection end of an infusion line to be connected to the connector shown in Fig. 3a;
Fig. 4 is a perspective view of an embodiment of an injection needle of the present invention;
Fig. 5a is a partially longitudinal sectional view of the injection needle of Fig. 4;
Fig. 5b is a partially longitudinal sectional view of an infusion tube to be connected to the injection needle of Fig. 5a;
Fig. 6 is a perspective view of an embodiment of an intravenous catheter of the present invention;
Fig. 7a is a partially longitudinal sectional view of the intravenous catheter of Fig. 6;
Fig. 7b is a partially longitudinal sectional view of an infusion tube to be connected to the intravenous catheter of Fig. 6;
Fig. 8 is a schematic view of an embodiment of an infusion tube of the present invention;
Fig. 9 is a partially longitudinal sectional view of the infusion tube of Fig. 8; and

Referring now to the accompanying drawings a connector of the present invention is explained.

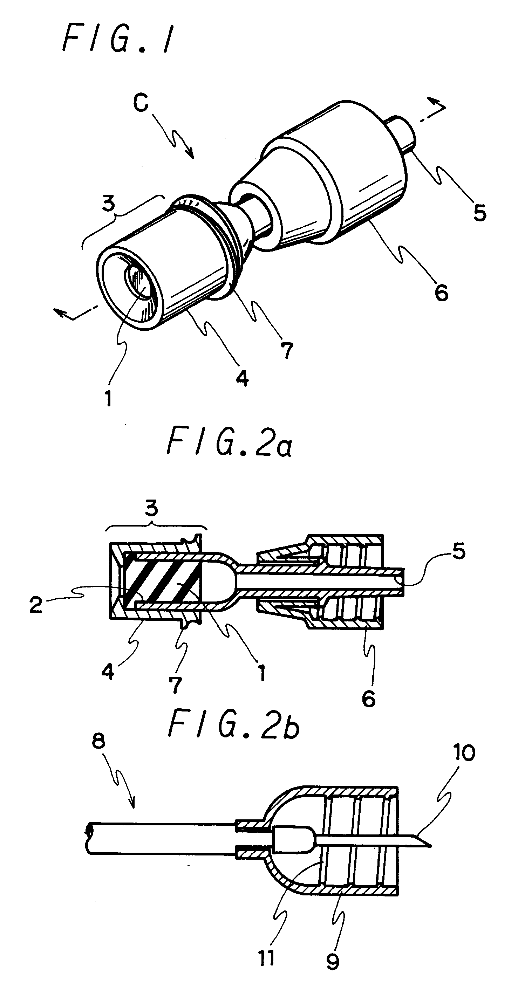

Fig. 1 is a perspective view of an embodiment of a connector of the present invention. The connector of Fig. 1 has a male screw as a connecting means and a connecting means comprising a female screw is provided at a fluid outlet.

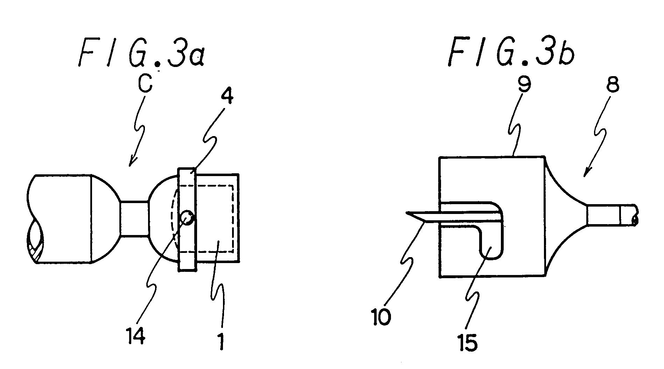

Fig. 2a is a sectional view taken along the line X-X of Fig. 1. Fig. 3a is a partially sectional view of another embodiment of a connector of the present invention, and Fig. 3b is a respectively partially secional view showing a connection end of an infusion line to be connected to the connector shown in Fig. 3a.

As shown in Figs. 1, 2a and 3a, a connector C of the present invention is a tubular connector having an injection site 3, and characterized in that a connecting means is provided on the external wall of the injection site 3 or on the external wall of the tubular body near the injection site 3. The connector C can be airtightly connected to an infusion line 8 having at its connection end a connecting means 9 of which shape corresponds to that of the connecting means 4 of Fig. 2a, Fig. 3a as shown in Figs. 2b, 3b. That is, the connector C and the infusion line 8 can be airtightly and firmly connected to each other by sticking a needle 10 of the infusion line 8 into the injection site 3 of the connector to make the connector C communicate with the infusion line 8 and then by combining both connecting means.

The connector of the present invention is hereinafter explained in detail mainly based on Figs. 1 and 2a.

The injection site 3 is used for infusing plural kinds of liquid drug into a patient by adding other liquid drugs from other infusion lines during fluid therapy. The injection site 3 comprises a tubular body (a part of the connector C excepting connecting means 4 and 6) having one or more fluid inlet 2 and a fluid outlet 5. A cap or plug made of rubber-like elastic material is liquidtightly put on at least one fluid inlet 2. In the case of a cap, it is put on the fluid inlet 2, while in the case of a plug, it is inserted into the fluid inlet 2 as shown in Fig. 2a. In that case, a means for supporting the cap or rubber plug 1 might be provided to prevent the detachment of the cap or rubber plug 1. In Fig. 2a, the connecting means 4 comprises a tubular portion functioning as a supporting means, and a male screw 7.

As described above the cap or plug is made of rubber-like elastic material. Concrete examples of the rubber-like elastic material are, for instance, natural rubber and synthetic rubber such as butadiene rubber, styrene-butadiene rubber, isoprene rubber, ethylene-propylene rubber, butyl rubber, chloroprene rubber, nitrile rubber, acrylic rubber, urethane rubber and silicone rubber. Among them, in particular, natural rubber, isoprene rubber, chloroprene rubber and silicone rubber are preferably used since they have small sticking resistance and good sealing property.

The connecting means 4 on the side of the fluid inlet 2 serves to connect the connector C to a device having a needle at its one end (hereafter representatively referred to as infusion line). The connecting means 4 alone does not function as a connector, but it cooperates with a connecting means 9 formed at the connecting end of the infusion line 8 and combines the connector with the infusion line. Accordingly the shape of the connecting means 9 at the connection end of the infusion line 8 varies depending on the shape of the connecting means 4. That is, the shape of the connecting means 4 of the connector C and that of the connecting means 9 of the infusion line 8 are supplementary to each other. Such relationship is found in, for example, a male screw 7 in Fig. 2a and a female screw 11 in Fig. 2b; a projection 14 in Fig. 3a and a slit 15 in Fig. 3b.

The connecting means 4 is provided on the external wall of the injection site 3 or on the external wall of a tubular portion near the injection site 3. When the connector C and the infusion line 8 is connected to each other after a needle 10 of the infusion line 8 is sticked into the plug 1 of the injection site 3, it is preferable that the connecting means 4 is so provided as to prevent the rotation of the needle in the rubber plug 1. Concretely speaking, the male screw 7 is preferably fixed to the connector C, i.e. adhered to or formed integrally with the connector C, when the connecting means 4 comprises the male screw 7 (see Fig. 2a). Further, when the connecting means 4 comprises a projection 14 or a slit 16, the projection 14 or slit 16 might be fixedly or rotatably provided at the connector (see Fig. 3a). In that case, it is necessary to employ, as a connecting means 9 of an infusion line 8, a rotatable female screw 11 for a fixed male screw 7; a rotatable slit 15 or projection 17 for a fixed projection 14 (see Figs. 2b, 3b).

A connecting means 6 on the side of the fluid outlet 5 is optionally employed, and therefore is not always necessary. The opening end of a tube might be directly inserted into the fluid outlet 5 and, if necessary, adhered thereto by means of adhesives and the like. In the case where the tube cannot be firmly connected to the connector C for reason of material, however, it is necessary to provide a connecting means 6 at the fluid outlet 5. By means of the connecting means 6, a tube having at its connection end a supplemental connecting means with the connecting means 6, for example, an intravenous catheter can be surely and securely connected to the connector.

As a material for a tubular portion of the connector C, it is preferable to employ sythetic resin having a resistance to liquid drug used as a solution for infusion. There can be preferably used, for example, polyethylene, polypropylene, hard vinyl chloride resin, acrylonitrile-butadiene-styrene copolymer, or styrene-acrylonitrile copolymer. Material for the connecting means 4 and 6 is not particularly limited in the present invention. Synthetic resin is generally employable, and polystyrene, polyamide, polyester, polycarbonate and polymethyl methacrylate can be preferably employed besides the materials described above as a material for the tubular portion of the connector.

Next a method of using a connector C of the present invention is explained.

In the case of a connector C shown in Fig. 2a having a male screw 7 as a connecting means 4, a needle 10 of the infusion line 8 having a female screw 11 as a connecting means as shown in Fig. 2b is sticked into a rubber plug 1 of an injection site 3 of the connector C. Then the rotation of the connecting means 9 of the infusion line 8 with one hand in the direction in which the connecting means 9 is screwed, while holding the connector C with other hand, gives the firm connection between the infusion line 8 and the connector C without causing the rotation of the needle 10 in the rubber plug 1.

In the case of a connector C having a projection 14 as a connecting means 4 as shown in Fig. 3a, a needle 10 of the infusion line 8 having a slit 15 as a connecting means as shown in Fig. 3b is sticked into a rubber plug 1 of an injection site 3 of the connector C. Then the rotation of the connecting means 9 of the infusion line 8 with one hand and the insertion of the projection into the slit 15, while holding the connector C with other hand, gives the firm connection between the infusion line 8 and the connector C.

Next there are explained in order three embodiments of an injection needle, an intravenous catheter, and an infusion tube which all adapt a connector of the present invention or its conception.

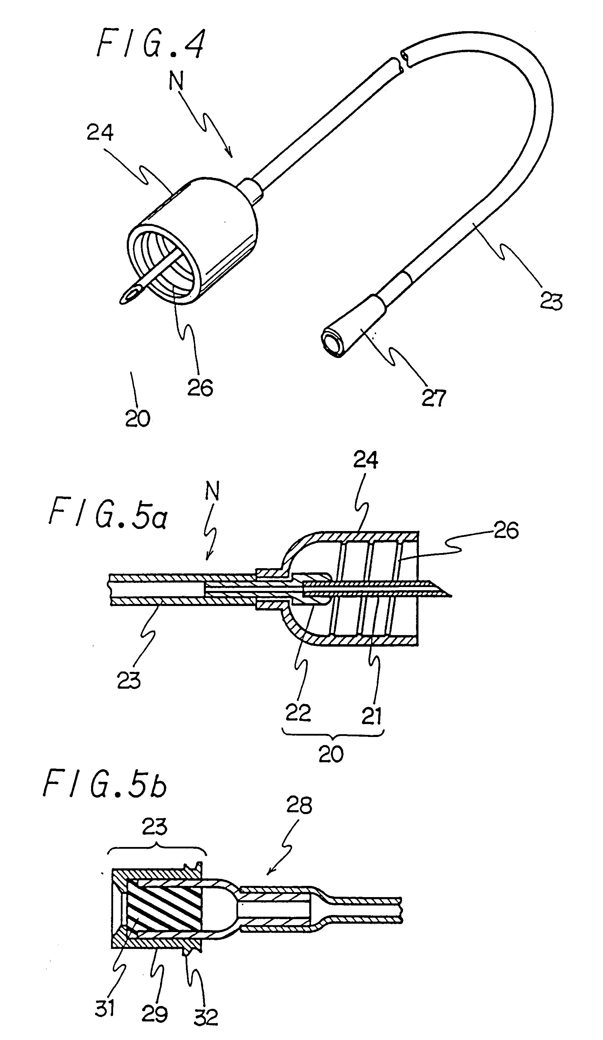

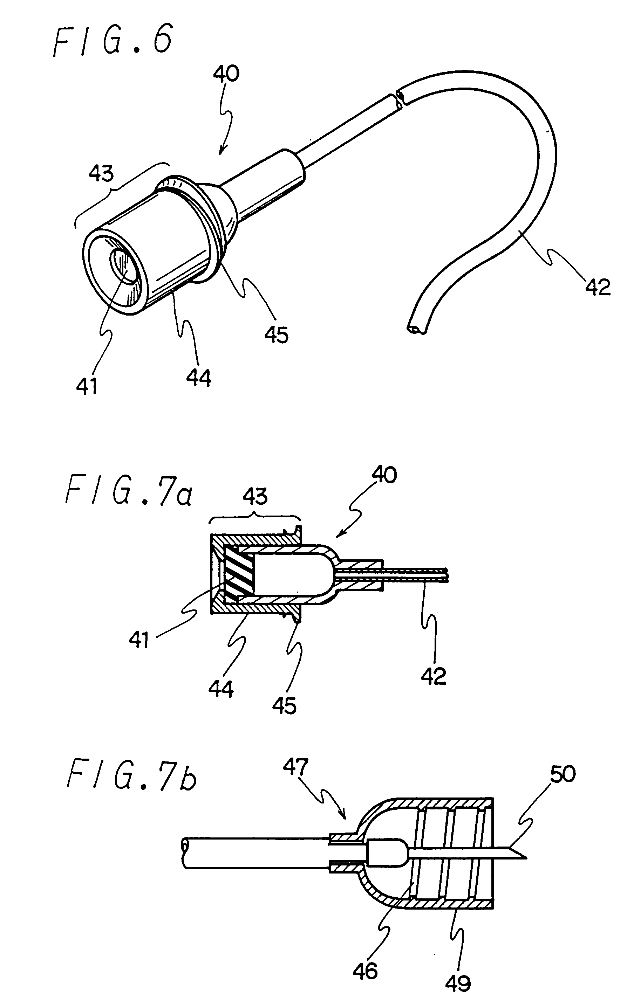

Fig. 4 is a perspective view of an embodiment of an injection needle of the present invention having a female screw as a connecting means. Fig. 5a is a partially longitudinal sectional view of the injection needle of Fig. 4, and Fig. 5b is a partially longitudinal sectional view of an infusion tube to be connected to the injection needle of Fig. 4.

As shown in Figs. 4 and 5a, an injection needle N of the present invention is characterized in that, in an injection needle having a tube 23 connected to the free end of a hub 22, a connecting means is provided at the hub 22. The injection needle N can be airtightly connected to an infusion tube 28 having, on the outer wall of an injection site 23 or on the external wall of the tubular body near the injection site 23, an connecting means 29 shown in Fig. 5b of which shape corresponds to that of the connecting means 24 of Fig. 5a. That is, the infusion needle N and the infusion tube can be airtightly and securely connected to each other by sticking a needle 20 of the injection needle N into a rubber plug 31 of the injection site 23 of the infusion tube 28 to make the injection needle N communicate with the infusion tube 28 and then by combining both connecting means.

The needle 20 comprises a canula 21 and a hub 22. In use, the needle 20 is sticked into the rubber plug 31 of the infusion tube 28 having an injection site 23 at its connection end and serves to make the injection needle N communicate with the infusion tube 28.

Material for the canula 21 and hub 22, and method of adhering them to each other to fabricate a needle 20 are not particularly limited in the present invention. Conventional material and method can be employed.

A tube 23 is generally made of soft synthetic resin such as soft vinyl chloride resin and polyethylene. The tube 23 is connected to a free end (an end opposite the canula 21) of the hub 22 directly or through other suitable connecting means. The other end of the tube 23 is formed into suitable shapes depending on the purpose or condition of use. That is, a connecting means might be provided at the other end of the tube 23, or the other end might be formed to provide an infusion line. In Fig. 4, a connector 27 for connecting an infusion line and the like is provided at the other end of the tube 23.

The connecting means 24 is provided at the hub 22. It is preferable that the connecting means 24 is so provided as to prevent the rotation of the needle 20 in the rubber plug 31 when the injection needle N is combined with the infusion tube 28 after the needle 20 is sticked into the rubber plug 31 of the injection site 23 of the infusion tube 28. For example, when employing a female screw 26 as a connecting means 24, it is preferable that the female screw 26 is rotatably provided at the hub 22 (see Fig. 5a). In that case, it is necessary to employ a male screw 12 as a connecting means 29 of the infusion tube 28 for the rotatable female screw 26 (see Fig. 5b).

As a material for the connecting means 24, 29 and rubber plug 31, the same material as in the connecting means and rubber plug of the connector described above can be employed.

A method of using an injection needle N of the present invention is explained.

In the case of an injection needle N shown in Fig. 5a having a female screw 26 as a connecing means 24, a needle 20 of the injection needle N is sticked into a rubber plug 31 of the injection site 23 of the infusion tube 28 having a male screw 32 as a connecting means 29 as shown in Fig. 5b. Then the rotation of the connecting means 24 of the injection needle N with one hand in the direction in which the connecting means 24 is screwed, while holding the infusion tube 28 with other hand, gives the firm connection between the injection needle N and the infusion tube 28.

Next an intravenous catheter adapting a connector of the present invention is explained.

Fig. 6 is a perspective view of an embodiment of an intravenous catheter of the present invention having a male screw as a connecting means. Fig. 7a is a partially longitudinal sectional view of the catheter of Fig. 6, and Fig. 7b is a partially longitudinal sectional view of an infusion line to be connected to the catheter of Fig. 6.

As shown in Figs. 6 and 7a, a catheter of the present invention comprises a tubular connector 40 whereto a tube 42 of a small diameter is connected, characterized in that the connector 40 has an injection site 43, and a connecting means 44 is provided on the external wall of the injection site 43 or on the external wall of a tubular portion near the injection site 43. The connector 40 can be airtightly connected to an infusion line 47 shown in Fig. 7b having at its connection end a connecting means 49 of which shape corresponds to that of the connecting means 44 of Fig. 7a. That is, the catheter and the infusion line 47 can be airtightly and securely connected to each other by sticking a needle 50 of the infusion line 47 into a rubber plug 41 of the injection site 43 of the catheter to make the catheter communicate with the infusion line 47 and then by combining both connecting means.

The injection site 43 is used for infusing plural kinds of liquid drug into a patient by adding other liquid drugs from other infusion lines during fluid therapy. The injection site 43 comprises a tubular body having one or more fluid inlet and a fluid outlet. A cap or plug made of rubber-like elastic material is liquidtightly put on at least one fluid inlet. In the case of a cap, it is put on the fluid inlet, while in the case of a plug, it is inserted into the fluid inlet as shown in Fig. 7a. In that case, a means for supporting the cap or rubber plug 41 might be provided to prevent the detachment of the cap or rubber plug 41. In Fig 7a, the connecting means 44 functions also as a supporting means.

As a material for the cap or plug, the same material as in the cap or plug of the above-mentioned connector can be employed.

The connecting means 44 serves to connect a catheter to a device to be connected (hereafter representatively referred to as infusion line), and the device has a needle at its connection end. The connecting means 44 alone does not function as a connector, but it cooperates with a connecting means 49 formed at the connection end of the infusion line 47 and combines the connector 40 with the infusion line 47. Accordingly the shape of the connecting means 49 at the connection end of the infusion line 47 varies depending on the shape of the connecting means 44. That is, the shape of the connecting means 44 of the catheter and that of the connecting means 49 of the infusion line 47 are supplementary to each other. Such relationship is found in, for example, a male screw 45 in Fig. 7a and a female screw 46 in Fig. 7b.

The connecting means 44 is provided on the external wall of the injection site 43 or on the external wall of a tubular portion near the injection site 43. It is preferable that the connecting means 44 is so provided as to prevent the rotation of the needle in the rubber plug 41 when the connector of the catheter and the infusion line 47 is connected to each other after a needle 10 of the infusion line 47 is sticked into the rubber plug 41 of the injection site 43. Concretely speaking, the male screw 45 is preferably fixed to the connector 40, i.e. adhered to or formed integrally with the connector 40, when the connecting means 4 comprises the male screw 45 (see Fig. 7a). In that case, it is necessary to employ, as a connecting means 49 of the infusion line 47, a rotatable female screw 46 for a fixed male screw 45 (see Fig. 7b).

A tube 42 is a main portion of the catheter. In use, an free end of the tube 42 is remained in vein of a patient. Fluid therapy is carried out by infusing liquid drug and the like into vein through the injection site 43. As material for the tube 42, soft sythetic resin can be employed, and the material is required to have bio-compatibility. Concrete examples of the preferably used material are, for example, vinyl chloride resin, polyurethane, and silicone rubber.

Next a method of using an intravenous catheter of the present invention is explained.

In the case of a catheter shown in Fig. 7a having a male screw 45 as a connecting means 44, a needle 50 of the infusion line 47 having a female screw 46 as a connecting means 49 as shown in Fig. 7b is sticked into a rubber plug 41 of an injection site 43 of the catheter. Then the rotation of the connecting means 49 of the infusion line 47 with one hand in the direction in which the connecting means 49 is screwed, while holding the catheter with other hand, gives the firm connection between the infusion line 47 and the catheter without causing the rotation of the needle 50 in the rubber plug 41.

Finally an infusion tube adapting a connector of the present invention is explained.

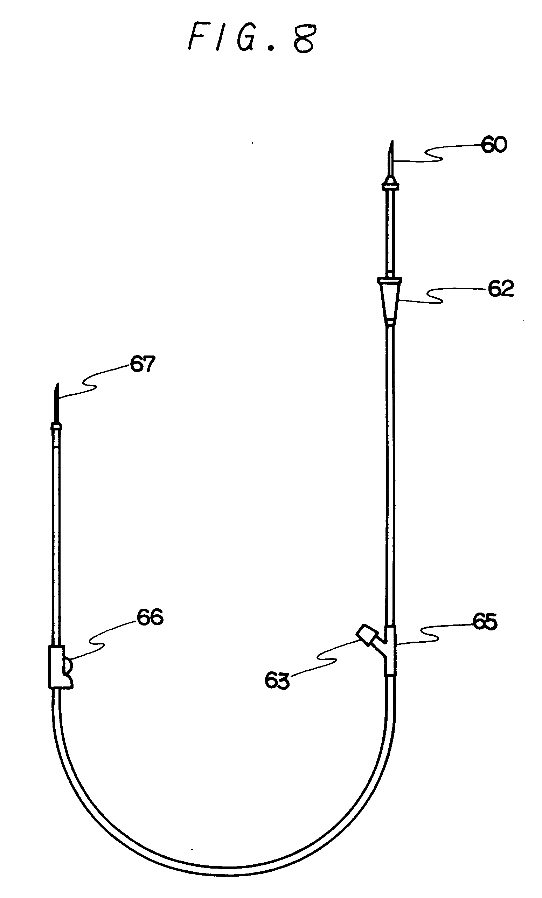

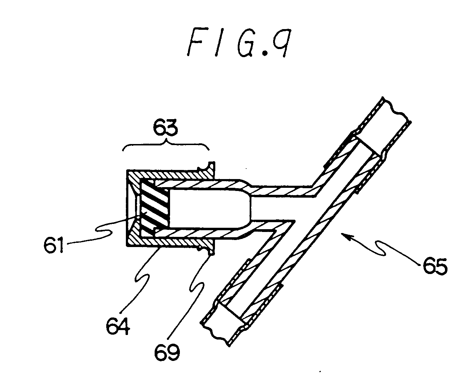

Fig. 8 is a perspective view of an embodiment of an infusion tube of the present invention having a male screw as a connecting means. Fig. 9 is a partially longitudinal sectional view of the infusion tube of Fig. 8.

As shown in Figs. 8 to 9, an infusion tube of the present invention comprises a spike needle or piercing spike (hereafter referred to as spike) 60, a drip chamber 62, an injection site 63, a flow-controlling means (a roller minicramp 66 is employed in Fig. 8), and an intravenous needle 67, and is characterized in that the injection site 63 is provided at a diverged tube of a tubular body (a Y-shaped tube 65 is employed in Fig 8), and that a connecting means 64 is provided on the external wall of the injection site 43 or on the external wall of a tubular portion near the injection site 63.

The connecting means 64 can be airtightly connected to an infusion line shown in Fig. 7b having at its connection end a connecting means of which shape corresponds to that of the connecting means of Fig. 9. That is, in the case of an infusion line of Fig. 7b, the infusion tube and the infusion line 47 can be air tightly and securely connected to each other by sticking a needle 50 of the infusion line 47 into the injection site 63 of the infusion line to make the infusion tube communicate with the infusion line 97 and then by combining both connecting means 64, 49.

The spike 60, drip chamber 62, Y-shaped tube 65 and intravenous needle 67 are connected to one another by means of, for example, a tube made of soft vinyl chloride resin, and constitutes an infusion line. The roller cramp 66 is provided between the Y-shaped tube 65 and intravenous needle 67.

The spike 60 is sticked into a plug of a container such as a bag in order to flow liquid drug and the like in the container into an infusion tube. The drip chamber 62 is used to monitor the priming of liquid drug and the like into the infusion tube and drip rate of liquid drug and the like flown. The Y-shaped tube 65 is a tubular body having a diverged route for introducing liquid drug and the like from other infusion routes. The intravenous needle 67 is sticked into vein of a patient to infuse liquid drug and the like into the vein of the patient.

The injection site 63 is used for infusing plural kinds of liquid drug into a patient by adding other liquid drugs from other infusion lines during fluid therapy. The injection site 63 comprises a tubular body having one or more fluid inlet and a fluid outlet. A cap or plug made of rubber-like elastic material is liquidtightly put on at least one fluid inlet. In the case of a cap, it is put on the diverged route, while in the case of a plug, it is inserted into the diverged route. In that case, a means for supporting the cap or rubber plug 61 might be provided to prevent the detachment of the cap or rubber plug 61. In Fig. 9, the connecting means 64 functions also as a supporting means.

As a material for the cap or plug, the same material as in the cap or plug of the above-mentioned connector can be employed.

The connecting means 64 serves to connect an infusion tube to an infusion line having a needle at its connection end.

The connecting means 64 alone does not function as a connector, but it cooperates with a connecting means 49 formed at the connection end of the infusion line and combines the infusion tube with the infusion line. Accordingly the shape of the connecting means at the connection end of the infusion line varies depending on the shape of the connecting means 44. That is, the shape of the connecting means 44 of the infusion tube and that of the connecting means 49 of the infusion line 8 are supplementary to each other. Such relationship is found in, for example, a male screw 69 in Fig. 9 and a female screw 46 in Fig. 7b. The connecting means 64 is provided on the external wall of the injection site 63 or on the external wall of a tubular portion near the injection site 63. It is preferable that the connecting means 64 is so provided as to prevent the rotation of the needle in the rubber plug 61 when the infusion tube and the infusion line is connected to each other after a needle of the infusion line 47 is sticked into the rubber plug 61 of the injection site 63. Concretely speaking, the male screw 69 is preferably fixed to the injection site 63 or the diverged route near the injection site 63, when the connecting means 64 comprises the male screw 69 (see Fig. 9). In that case, it is necessary to employ, as a connecting means 49 of an infusion line 47, a rotatable female screw 46 for a fixed male screw 69 of the infusion tube (see Fig. 7b).

As a tubular body having a diverged route, various kinds of tubular bodies such as T-shaped tube, crossed tube and tube having a plurality of diverged routes are employable beside the Y-shaped tube 65. Among them, a Y-shaped tube or T-shaped tube is generally employed.

Next a method of using infusion tube of the present invention is explained.

In the case of a infusion tube shown in Fig. 9 having a male screw 69 as a connecting means 64, a needle 50 of the infusion line 47 having a female screw 46 as a connecting means 49 as shown in Fig. 7b is sticked into a rubber plug 61 of an injection site 63 of the infusion tube. Then the rotation of the connecting means 49 of the infusion line 47 with one hand in the direction in which the connecting means 49 is screwed, while holding the infusion tube with other hand, gives the firm connection between the infusion line 47 and the infusion tube without causing the rotation of the needle 50 in the rubber plug 61.

The connector of the present invention is applicable to other devices such as blood circuit and solution infusion device besides an infusion needle, intravenous catheter and infusion tube explained hereinbefore. Though male screws and female screws are employed in the above explanations for an injection needle, intravenous catheter and infusion tube, pin-shaped or stake-shaped projections are off course employable.

As is clear from the above explanation, the following effects can be obtained by the use of a connector of the present invention.
(1) Sheets or clothes of a patient is not soiled with liquid drug since the needle of an infusion line is not pulled out during treatment.
(2) Infection by the air through an injection site can be perfectly avoided by the structure enabling airtight connection.

## Claims

1. Infusion assembly, comprising
a connector (C) having a tubular body with one or more fluid inlet(s) (2) and fluid outlet(s) (5), a cap or plug (1) made of rubber-like elastic material being liquidtightly fitted into at least one fluid inlet (2) to form an injection site (3), a connecting means (4) being provided on the external wall of the injection site or on the external wall of the tubular body near the injection site, and
an injection needle (8,N) having a cannula (21) attached to one end of a hub (22), a tube (23) connected to the other end of the hub (22) directly or through a connector and a cap-shaped or hood-like connecting means (9,24) which is rotatably provided at the hub (22) and surrounds the hub, the free end of the cannula (21) projecting out of the connecting means (9,24) of said injection needle (8,N) to permit said cannula (21) to pierce the cap or plug (1) prior to engagement of the connecting means (4;9,24) of said connector (C) and said injection needle (8,N),
the shape of the connecting means (9,24) of said injection needle (8,N) corresponding to the shape of the connecting means (4) of said connector (C), said injection needle (8,N) being sticked into the cap or plug (1) of said connector (C) and said connecting means (4;9,24) being engaged.

2. Infusion assembly of claim 1, wherein the connecting means (4) of said connector (C) comprises a male screw (7) and the connecting means (9,24) of said injection needle (8,N) comprises a female screw (11).

3. Infusion assembly of claim 1, wherein the connecting means (9,24) of said injection needle (8,N) comprises an approximately L-shaped connecting slit (15), and wherein the connecting means (4) of said connector (C) comprises a pin-shaped or stake-shaped projection (14).

4. Infusion assembly of one of claims 1 to 3, wherein a connecting means (6) is also provided at the fluid outlet (5) of said connector (C).

5. An intravenous catheter comprising an infusion assembly as claimed in one of claims 1 to 4, wherein a tube (42) of small diameter is provided, said tube (42) being liquidtightly connected to the fluid outlet (5) of said connector (C,40).

6. An infusion tube comprising
a spike needle (60), a drip chamber (62), a flow-controlling means (66), an intravenous needle (67),
a tubular body (65) having a diverged route, a cap or plug (61) made of rubber-like elastic material being liquidtightly fitted into the diverged route to form an injection site (63), a connecting means (64) being provided on the external wall of the injection site (63) or on the external wall of a tubular body near the injection site (63), and
an injection needle (8,N) having a cannula (21) attached to one end of a hub (22), a tube (23) connected to the other end of the hub (22) directly or through a connector and a cap-shaped or hood-like connecting means (9,24) which is rotatably provided at the hub (22) and surrounds the hub, the free end of said cannula (21) projecting out of the connecting means (9,24) of said injection needle (8,N) to permit said cannula (21) to pierce the cap or plug (61) prior to engagement of the connecting means (64;9,24) of said tubular body (65) and said injection needle (8,N),
the shape of the connecting means (9,24) of said injection needle (8,N) corresponding to the shape of the connecting means (64) of said tubular body (65), said injection needle (8,N) being sticked into the cap or plug (61) of said tubular body (65) and said connecting means (64;9,24) being engaged.

## Patentansprüche

1. Eine Infusionsleitung, welche folgendes aufweist:
ein Verbindungsstück (C) enthaltend einen röhrenförmigen Körper mit einem oder mehreren Fluideinlässen (2) und Fluidauslässen (5), eine Kappe oder einen Stöpsel (1) aus einem elastischen gummiartigen Material, welche leckdicht in mindestens einem Fluideinlaß (2) eingepaßt ist, um eine Injektionsstelle (3) zu bilden, außerdem enthaltend ein Verbindungsmittel (4), welches auf der Außenwand der Injektionsstelle oder auf der Außenwand des röhrenförmigen Körpers in der Nähe der Injektionsstelle befestigt ist, und mit einer Injektionsnadel (8, N) mit einer an einem Ende einer Nabe (22) befestigten Kanüle (21) und mit einem Schlauch (23), welcher entweder direkt oder mit Hilfe eines Verbindungsstückes und einem kappenartigen oder haubenartigen Verbindungsmittel (9, 24), welches drehbar an der Habe (22) befestigt ist und die Habe umgibt, an dem anderen Ende der Habe (22) befestigt ist, wobei das freie Ende der Kanüle (21) aus dem Verbindungsmittel (9, 24) der Injektionsnadel (8, N) herausragt, damit die Kanüle (21) die Kappe oder den Stöpsel (1) durchbohren kann, bevor das Verbindungsmittel (4; 9, 24) des Verbindungsstückes (C) mit der Injektionsnadel (8, N) in Eingriff tritt, wobei die Form des Verbindungsmittels (9, 24) der Injektionsnadel (8, N) der Form des Verbindungsmittels (4) des Verbindungsstückes (C) entspricht, und die Injektionsnadel (8, N) in die Kappe oder den Stöpsel (1) des Verbindungsstückes (C) eingestochen wird und mit dem Verbindungsmittel (4; 9, 24) in Eingriff tritt.

2. Infusionsleitung nach Anspruch 1, in der das Verbindungsmittel (4) des Verbindungsstückes (C) eine Schraubenspindel (7) und das Verbindungsmittel (9, 24) der Injketionsnadel (8, N) eine Steckerbuchse (11) aufweist.

3. Infusionsleitung nach Anspruch 1, in der das Verbindungsmittel (9, 24) der Injektionsnadel (8, N) einen etwa L-förmigen Verbindungsschlitz (15) aufweist, und in der das Verbindungsmittel (4) des Verbindungstückes (C) eine stiftartige oder pflockartige Auskragung (14) aufweist.

4. Infusionsvorrichtung nach einem der Ansprüche 1 bis 3, in der ein Verbindungsmittel (6) auch an dem Fluidauslaß (5) des Verbindungsstückes (C) vorgesehen ist.

5. Intravenöser Katheter mit einer Infusionsleitung nach einem der Ansprüche 1 bis 4, in dem ein Schlauch (42) mit einem kleinen Durchmesser vorgesehen ist und dieser Schlauch (42) leckdicht an dem Fluidauslaß (5) des Verbindungsstückes (C, 40) befestigt ist.

6. Ein Infusionsschlauch, der folgendes enthält:
eine Nadel (60), eine Tropfkammer (62), eine Durchflußkontrolle (66), eine intravenöse Nadel (67), sowie einen röhrenförmigen Körper (65) mit einer divergierenden Bahn, einer Kappe oder einem Stöpsel (61) aus einem elastischen gummiartigen Material, welcher leckdicht in die divergierende Bahn eingesetzt ist, um so eine Injektionsstelle (63) zu bilden, ein Verbindungsmittel (64), welches auf der Außenwand der Injektionsstelle (63) oder auf der Außenwand des röhrenförmigen Körpers in der Nähe der Injektionsstelle (63) angeordnet ist, und mit einer Injektionsnadel (8, N) mit einer Kanüle (21), welche an einem Ende einer Nabe (22) befestigt ist, während ein Schlauch (23) mit dem anderen Ende der Nabe (22) entweder direkt oder über ein Verbindungsstück und ein kappenartiges oder haubenartiges Verbindungsmittel (9, 24) verbunden ist, welches drehbar an der Nabe (22) vorgesehen ist und diese Nabe umgibt, wobei das freie Ende der Kanüle (21) aus dem Verbindungsmittel (9, 24) der Injektionsnadel (8, N) herausragt, damit die Kanüle (21) die Kappe oder den Stöpsel (61) durchbohren kann, bevor das Verbindungsmittel (64; 9, 24) des röhrenförmigen Körpers (65) mit der Injektionsnadel (8, N) in Eingriff tritt, wobei die Form des Verbindungsmittels (9, 24) der Injektionsnadel (8, N) der Form des Verbindungsmittels (64) des röhrenförmigen Körpers (65) entspricht und die Injektionsnadel (8, N) in die Kappe oder den Stöpsel (61) des röhrenförmigen Körpers (65) eingestochen wird und mit dem Verbindungsmittel (64; 9, 24) in Eingriff tritt.

## Revendications

1. Assemblage de perfusion, comprenant
un élément de raccordement (C) comportant un corps tubulaire avec une ou plusieurs entrée(s) de fluide (2) et sortie(s) de fluide (5), un couvercle ou tampon (1) réalisé en un matériau élastique tel que du caoutchouc étant adapté de manière étanche aux liquides dans au moins une entrée de fluide (2) pour former un emplacement d'injection (3), un moyen de raccordement (4) étant disposé sur la paroi externe de l'emplacement d'injection ou sur la paroi externe du corps tubulaire à proximité de l'emplacement d'injection, et
une aiguille d'injection (8, N) comportant une canule (21) fixée à une extrémité d'un moyeu (22), un tube (23) raccordé à l'autre extrémité du moyeu (22) directement ou par l'intermédiaire d'un élément de raccordement et un moyen de raccordement (9, 24) en forme de couvercle ou en forme de capuchon, qui est disposé de manière rotative au niveau du moyeu (22) et qui entoure le moyeu, l'extrémité libre de la canule (21) faisant saillie à l'extérieur du moyen de raccordement (9, 24) de ladite aiguille d'injection (8, N) pour permettre à ladite canule (21) de percer le couvercle ou le tampon (1) avant la coopération du moyen de raccordement (4; 9, 24) dudit élément de raccordement (C) et de ladite aiguille d'injection (8, N),
la forme du moyen de raccordement (9, 24) de ladite aiguille d'injection (8, N) correspondant à la forme du moyen de raccordement (4) dudit élément de raccordement (C), ladite aiguille d'injection (8, N) étant enfoncée dans le couvercle ou bouchon (1) dudit élément de raccordement (C) et ledit moyen de raccordement (4; 9, 24) étant engagés l'un sur l'autre.

2. Assemblage de perfusion selon la revendication 1, dans lequel le moyen de raccordement (4) dudit élément de raccordement (C) comprend une vis mâle (7) et le moyen de raccordement (9, 24) de ladite aiguille d'injection (8, N) comprend une vis femelle (11).

3. Assemblage de perfusion selon la revendication 1, dans lequel le moyen de raccordement (9, 24) de ladite aiguille d'injection (8, N) comprend une fente de raccordement (15) approximativement en forme de L, et dans lequel le moyen de raccordement (4) dudit élément de raccordement (C) comprend une saillie (14) en forme d'épingle ou en forme de pique.

4. Assemblage de perfusion selon l'une des revendications 1 à 3, dans lequel un moyen de raccordement (6) est aussi disposé au niveau de la sortie de fluide (5) dudit élément de raccordement (C).

5. Cathéter intraveineux comprenant un assemblage de perfusion selon l'une des revendications 1 à 4, dans lequel un tube (42) de petit diamètre est disposé, ledit tube (42) étant raccordé de manière étanche aux liquides à la sortie de fluide (5) dudit élément de raccordement (C, 40).

6. Tube de perfusion comprenant
une aiguille à pointe (60), une chambre d'écoulement goutte à goutte (62), un moyen (66) de commande d'écoulement, une aiguille intraveineuse (67),
un corps tubulaire (65) ayant un trajet divergent, un couvercle ou tampon (61) réalisé en un matériau élastique tel que du caoutchouc, étant adapté de manière étanche aux liquides dans le trajet divergent pour former un emplacement d'injection (63), un moyen de raccordement (64) étant disposé sur la paroi externe de l'emplacement d'injection (63) ou sur la paroi externe d'un corps tubulaire à proximité de l'emplacement d' injection (63), et
une aiguille d'injection (8, N) ayant une canule (21) fixée à une extrémité d'un moyeu (22), un tube (23) raccordé à l'autre extrémité du moyeu (22) directement ou par l'intermédiaire d'un élément de raccordement et un moyen de raccordement (9, 24) en forme de couvercle ou en forme de capuchon qui est disposé de manière rotative au niveau du moyeu (22) et qui entoure le moyeu, l'extrémité libre de ladite canule (21) faisant saillie à l'extérieur du moyen de raccordement (9, 24) de ladite aiguille d'injection (8, N) pour permettre à ladite canule (21) de percer le couvercle ou le bouchon (61) avant la coopération du moyen de raccordement (64; 9, 24) dudit corps tubulaire (65) et de ladite aiguille d'injection (8, N),
la forme du moyen de raccordement (9, 24) de ladite aiguille d'injection (8, N) correspondant à la forme du moyen de raccordement (64) dudit corps tubulaire (65), ladite aiguille d'injection (8, N) étant enfoncée dans le couvercle ou bouchon (61) dudit corps tubulaire (65) et lesdits moyens de raccordement (64; 9, 24) étant engagés l'un sur l'autre.
